# FASCICULE DE BREVET EUROPEEN

(11) **EP 2 802 875 B1**
(45) Date de publication et mention de la délivrance du brevet: **31.03.2021**
(21) Numéro de dépôt: 13701855.2
(22) Date de dépôt: 09.01.2013
(51) Int. Cl.: C12C 1/00, G01N 33/543, C12M 1/34, C12Q 1/04, C12Q 1/00, B01L 3/00, G01N 33/569

(54) **PROCEDE DE DETECTION ET D'IDENTIFICATION DIRECTE PAR VOIE OPTIQUE D'UN MICROORGANISME DANS UN ECHANTILLON BIOLOGIQUE**
VERFAHREN ZUM DIREKTEN NACHWEIS UND ZUR IDENTIFIZIERUNG VON MIKROORGANISMEN IN EINER BIOLOGISCHEN PROBE DURCH OPTISCHE MITTEL
METHOD FOR DIRECTLY DETECTING AND IDENTIFYING A MICROORGANISM IN A BIOLOGICAL SAMPLE VIA OPTICAL MEANS

(30) Priorité: 10.01.2012 FR 1250249
(43) Date de publication de la demande: 19.11.2014
(73) Titulaire: bioMérieux, 69280 Marcy l'Etoile (FR)
(72) Inventeur: RAYMOND, Jean-Claude, F-69690 Bessenay (FR); MOSTICONE, David, 69280 Sainte Consorce (FR); VIMONT, Antoine, 69630 Chaponost (FR); BARIL, Florent, 01800 Saint Jean de Niost (FR); FLANDROIX, Jean-Pierre, F-69003 Lyon (FR); JUNILLON, Thomas, 69007 Lyon (FR); MALLEN, Benoit, 69160 Tassin la Demi-Lune (FR)
(86) Numéro de dépôt international: PCT/FR2013/050051
(87) Numéro de publication internationale: WO 2013/104864

(56) Documents cités:
- EP-A2- 0 446 972
- WO-A1-96/39533
- WO-A1-2007/106936
- WO-A1-2012/004540
- FR-A1- 2 928 656
- US-A1- 2009 081 766
- VIMONT ET AL: "Growth of Shiga-Toxin producing Escherichia coli (STEC) and bovine feces background microflora in various enrichment protocols", VETERINARY MICROBIOLOGY, ELSEVIER BV, NL, vol. 123, no. 1-3, 20 juillet 2007 (2007-07-20), pages 274-281, XP022114804, ISSN: 0378-1135, DOI: 10.1016/J.VETMIC.2007.02.003
- KIM N ET AL: "Application of a flow-type antibody sensor to the detection of Escherichia coli in various foods", BIOSENSORS AND BIOELECTRONICS, ELSEVIER BV, NL, vol. 18, no. 9, 15 août 2003 (2003-08-15), pages 1101-1107, XP002604415, ISSN: 0956-5663
- YANG ET AL: "Electrical/electrochemical impedance for rapid detection of foodborne pathogenic bacteria", BIOTECHNOLOGY ADVANCES, ELSEVIER PUBLISHING, BARKING, GB, vol. 26, no. 2, 12 novembre 2007 (2007-11-12), pages 135-150, XP022426821, ISSN: 0734-9750, DOI: 10.1016/J.BIOTECHADV.2007.10.003

## Description

La présente invention concerne, de façon générale, le domaine de l'analyse par exemple l'analyse biologique. Plus spécifiquement, la présente invention concerne un procédé de détection et d'identification directe d'au moins un microorganisme par voie optique dans un échantillon biologique, éventuellement enrichi.

L'analyse microbiologique nécessite des techniques précises et dont le temps d'obtention du résultat doit être le plus court possible.

Dans le domaine médical, il est nécessaire de prévoir et diagnostiquer le risque infectieux : plus le diagnostic est rapide et précis, plus la prise en charge des malades est efficace et le risque de transmission minimisé. L'approche est similaire pour la santé animale.

Dans le domaine agroalimentaire, la problématique est identique. Elle distingue cependant :
- les microorganismes pathogènes et leurs toxines dont la recherche s'applique aux matières premières, produits intermédiaires, produits finis commercialisés,
- les microorganismes non pathogènes, utilisés comme indicateurs-qualité du processus de production, des matières premières aux produits finis, tout au long de la chaîne, et
- les bactéries d'intérêt technologique telles les ferments.

La détection rapide et précise de contaminations présumées permet de les contrôler et d'engager ainsi des actions correctives.

Techniquement, l'analyse microbiologique peut mettre en œuvre une ou plusieurs phases de pré-enrichissement et/ou d'enrichissement, une ou plusieurs phases de détection, une ou plusieurs phases d'énumération des microorganismes. Pour des applications particulières telles le contrôle microbiologique agroalimentaire, une phase de confirmation peut également être requise, afin de répondre aux normes en vigueur dans ce domaine.

A l'heure actuelle, il n'existe pas de méthode pour détecter un microorganisme cible dans une quantité d'échantillon initiale importante, sans faire appel à une étape d'enrichissement.

La phase d'enrichissement fait appel à des milieux de culture, sélectifs ou non, qui ont pour but de promouvoir la croissance des microorganismes cibles dans les échantillons biologiques ou environnementaux, tout en limitant la croissance des flores non cibles. Les milieux sont souvent utilisés dans des conteneurs du type sac en plastique stérile, dans lesquels ils sont mis en contact avec les échantillons alimentaires ou environnementaux, à fins de remise en suspension et enrichissement des microorganismes recherchés. Cette phase est nécessaire afin de répondre aux besoins qui est de révéler la présence initiale potentielle d'au moins un microorganisme cible dans une quantité d'échantillon très variable et éventuellement très grande, e.g. 25 grammes (g) à 375 g dilué dans 225 à 3375 millilitres (mL) dans le milieu de culture. A l'issue de cette étape d'enrichissement, un aliquote (de 5 microlitres (µl) à 5 mL) est prélevé pour mettre en œuvre l'étape de détection des microorganismes cibles. Or, dans cet aliquote, il est nécessaire de disposer d'une quantité suffisante de microorganismes cibles pour s'assurer de leur détection systématique. Une étape d'enrichissement secondaire ou subculture peut alors être nécessaire. Vimont et al. (veterinary microbiology, Elsevier BV, NL, vol 123 no 1-3, 20 juillet 2007, page 274-281) décrit l'étude de différentes conditions d'enrichissement permettant la croissance simultanée d'Escherichia coli productrices de shiga-toxines et de la microflore bactérienne habituellement présente dans les fèces bovins.

La phase de détection se base historiquement sur la culture des microorganismes sur milieux gélosés, pour la mise en évidence des caractères métaboliques des microorganismes recherchés. On utilise classiquement des substrats enzymatiques spécifiques. Ces substrats enzymatiques sont généralement composés de deux parties, une première partie spécifique de l'activité enzymatique à révéler, appelée également partie cible, et une seconde partie faisant office de marqueur, appelée partie marqueur, généralement constituée par un chromophore ou un fluorophore. Par le choix de ces substrats, selon qu'il y a réaction ou non, il est possible de caractériser la nature d'un microorganisme ou de discriminer différents groupes de microorganismes. Ainsi, l'apparition ou la disparition d'une coloration ou d'une fluorescence sera la signature d'un genre ou d'un type de microorganismes. A cet égard, l'utilisation de milieux chromogènes permet la détection et l'identification simultanées des germes recherchés. Elle simplifie le processus et diminue sensiblement le délai d'obtention du résultat. On citera à titre d'exemple concret les milieux ChromID® de la demanderesse. Ces milieux chromogènes sont basés sur la détection de caractères métaboliques spécifiques des germes recherchés comme par exemple l'activité enzymatique bêta-glucuronidase pour *Escherichia coli.*

Les immuno-essais constituent une autre des technologies utilisées pour le test de détection. Ils font appel aux caractéristiques immunogènes des microorganismes recherchés. De façon non exhaustive, on peut citer les techniques d'immunofluorescence, les techniques ELISA (Enzyme Linked Immuno Sorbent Assay), par compétition ou de type sandwich. Ces techniques font appel à une étape de révélation dite indirecte qui met en œuvre un anticorps secondaire conjugué à une enzyme pour une détection ultérieure via un substrat spécifique de cette dernière.

Le document EP-B-1 440 316 décrit par exemple un dispositif permettant la détection des microorganismes. Ce dispositif est constitué d'un support solide sur lequel sont fixés des partenaires de captures spécifiques des microorganismes cibles, tels que des anticorps. Le support de capture est ensuite placé dans différents conteneurs comprenant l'échantillon à analyser et les différents réactifs pour réaliser une réaction ELISA. Cette étape de révélation dite indirecte implique (suite à l'étape d'enrichissement) alors la réalisation de diverses étapes de traitement (prélèvement, chauffage, centrifugation, lavages....) de l'échantillon avant l'étape de screening/détection, qui ont pour conséquence la complexification du protocole opératoire, la dégradation de la praticité de l'analyse et l'accroissement du temps de rendu des résultats.

Enfin, les techniques de biologie moléculaire, basées sur les caractères génomiques des microorganismes recherchés, sont également mise en œuvre pour détecter et identifier les microorganismes cibles. On citera à titre d'exemple les techniques d'amplification classiques telles la PCR (Polymerase Chain Reaction) et la NASBA (Nucleic Acid Sequence Based Amplification), qui peuvent être couplées à des techniques de détection en temps réel connues de l'homme du métier. Néanmoins, ces techniques nécessitent une étape lourde de préparation des échantillons, consistant à isoler les microorganismes, les lyser afin de libérer les acides nucléiques et enfin purifier ces derniers. Ceci a également un effet direct sur la complexification du protocole opératoire, la dégradation de la praticité de l'analyse et l'accroissement du temps de rendu des résultats.

La phase de confirmation, quant à elle, est plus particulièrement attachée à l'analyse microbiologique dans le domaine agroalimentaire. En effet, lorsque le résultat des méthodes développées précédemment est positif, il est nécessaire de confirmer la présence du pathogène recherché. Ceci impose un test complémentaire et l'utilisation d'un principe de détection différent de celui utilisé lors de la première analyse. Les techniques décrites *supra* sont à loisir utilisées pour la confirmation.

L'identification complète et précise d'un microorganisme dans un échantillon nécessite donc l'enchaînement de plusieurs étapes : enrichissement, éventuellement subculture, détection et confirmation. La standardisation des tests utilisés en routine a permis l'automatisation des méthodes de détection qui restent cependant longues à mettre en œuvre. Un inconvénient de l'état de la technique est en effet que ces étapes sont réalisées de manière séquentielle et nécessitent un grand nombre de manipulations chronophages, impactant ainsi le temps nécessaire au rendu des résultats.

Le document FR 2 928 656 décrit un procédé de détection en temps réel de microorganismes dans un milieu de culture par agglutination.
Le document WO2007/106936 décrit un détecteur électrochimique permettant de détecter un analyte.
Les documents EP 0 446 972 et WO96/39533 décrivent un instrument et un consomable permettant la détection de microorganismes dans un échantillon, ledit consommable comprenant un milieu de culture et un « sensor » de pH ou de concentration en CO₂

Eu égard aux problèmes techniques soulevés par l'état de la technique considéré ci-dessus, un des objectifs essentiels de la présente invention est de fournir un procédé simplifié pour la détection, l'identification et la confirmation des microorganismes présents dans des échantillons, en particulier agroalimentaires.

Un autre objectif de la présente invention est de fournir un procédé pour la détection et l'identification des microorganismes, qui permet de diminuer le temps et le coût nécessaires à l'analyse de l'échantillon

Ci-après, les terminologies, tel que « objectif(s) » et/ou « mode de réalisation » se rapportent à la divulgation et ne font partie de l'invention que si elles relèvent du champ d'application des revendications. La portée de la protection est définie seulement par les revendications annexées.

Ces objectifs parmi d'autres sont résolus par la présente invention, qui concerne en premier lieu un procédé de détection, d'au moins un microorganisme, ledit procédé comprenant essentiellement les étapes suivantes :
a) Mettre en contact dans un premier conteneur, ledit échantillon avec au moins un milieu de culture,
b) Placer ledit premier conteneur dans des conditions aptes à permettre la croissance du ou des microorganisme(s), pendant 6 à 48h ;
c) Mettre en contact tout ou partie du mélange constitué par l'échantillon et le milieu de culture avec un milieu réactionnel et un support de capture monobloc du ou desdit(s) microorganisme(s) dans un deuxième conteneur, ledit milieu réactionnel comportant des nutriments, un système sélectif permettant d'accroitre la population de bactéries cibles, un moyen de révélation du ou desdits microorganisme(s) choisi parmi les colorants et les composés fluorescents;
d) Détecter à l'intérieur dudit premier ou deuxième conteneur, la présence du ou des microorganisme(s) révélé(s) par le moyen de révélation et fixé(s) sur le support de capture, la détection étant réalisée en temps réel.

Selon l'invention, la méthode comporte une étape intermédiaire c') consistant à placer le deuxième conteneur dans des conditions aptes à permettre la croissance du ou des microorganisme(s).

Selon un mode de réalisation particulier, le procédé selon l'invention comporte une étape supplémentaire e) consistant à confirmer la détection du ou des microorganismes détecté(s). Préférentiellement, l'étape e) de confirmation est réalisée à l'aide d'un moyen de détection, identique ou différent du moyen de révélation utilisé pour la détection.

Avantageusement, au moins un partenaire de liaison, spécifique ou non spécifique, du ou des microorganismes est fixé sur le support de capture. Selon un mode de réalisation préférentiel de l'invention, le partenaire de liaison spécifique est pris dans le groupe comprenant : les anticorps, les fragments Fab, les fragments Fab', les aptamères, les protéines de phages recombinantes ou non, les phages ou tout autre ligand bien connu de l'homme du métier.

Le support de capture peut être tout support apte à permettre la révélation des microorganismes. On citera les supports monobloc, éventuellement poreux. Il peut être tout simplement un support inerte, tel une plaque de matériau plastique ou de la fibre de verre. Selon l'invention, le support est un support monobloc. Le support de capture peut être avantageusement sensibilisé avec un partenaire de liaison, éventuellement spécifique. Le support de capture peut être également un support monobloc compressible.

Avantageusement, le support peut être protégé par un film protecteur.

Selon un autre mode de réalisation particulier, il est possible de réaliser la détection et la confirmation avec la même technologie.

Selon l'invention, la détection du ou des microorganisme(s) est réalisée en temps réel.

Selon un mode particulier du procédé selon l'invention, le premier et/ou le deuxième conteneur est un sac d'homogénéisation. Il peut également s'agir de conteneurs rigides tels que flacons, bouteilles ou piluliers.

Selon un autre mode de réalisation particulier du procédé selon l'invention, l'étape de détection peut être réaliser à l'aide d'un dispositif de lecture. Un tel dispositif de lecture peut par exemple être constituée par une caméra pointant sur le support de capture, permettant l'enregistrement ou l'analyse d'images dudit support.

Les buts et avantages de la présente invention seront mieux compris à la lumière de la description détaillée qui suit, en lien avec le dessin dans lequel :
- La figure 1 est une représentation schématique des différentes étapes du procédé selon un premier mode de réalisation de l'invention.
- La figure 2 est une représentation schématique d'un support de capture sensibilisé.
- La figure 3 est une représentation schématique du support de capture sensibilisé, représenté sur la figure 2, après analyse dont le résultat est positif.

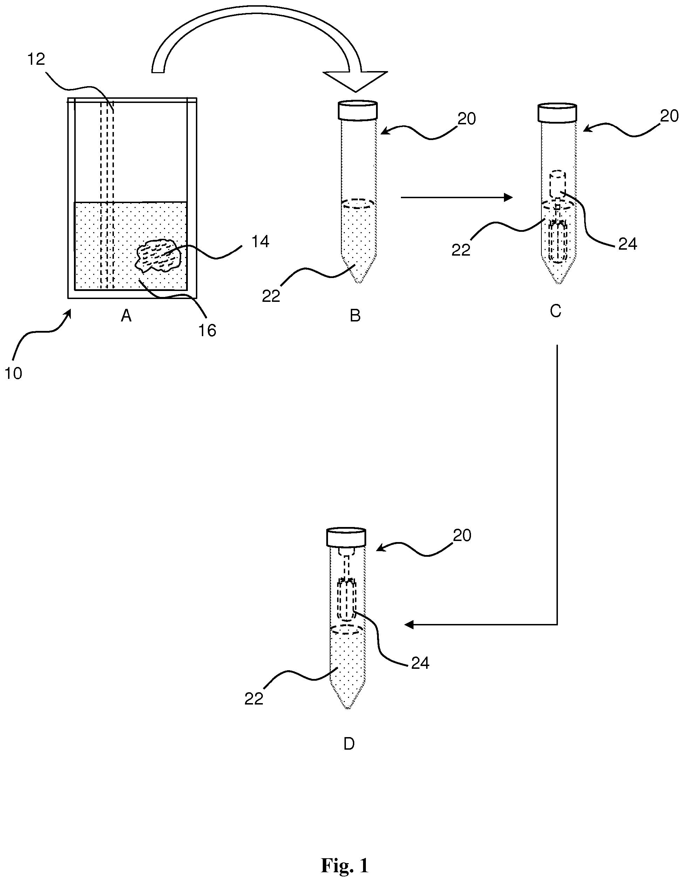

Selon un premier mode de réalisation de la présente divulgation le procédé de détection, voire d'identification de microorganisme consiste à mettre en œuvre un sac d'homogénéisation en plastique et stérile, classiquement appelé sac Stomacher®. Un tel sac est référencé 10 en figure 1A. Ce sac 10 est constitué de deux feuilles de plastique sensiblement rectangulaires, solidarisées l'une à l'autre par trois de leurs côtés, de manière à définir un espace intérieur destiné à recevoir le milieu de culture et l'échantillon à analyser. Il comporte accessoirement un filtre 12 de forme sensiblement rectangulaire et relié aux feuilles par un côté, séparant l'espace intérieur en deux.

Durant l'étape A, le sac d'homogénéisation 10, fermé, est incubé avec un échantillon alimentaire 14, constitué ici par un échantillon de fromage au lait cru. Cet échantillon alimentaire 14 est plongé dans un milieu d'enrichissement 16, qui peut être sélectif ou non. L'incubation peut être réalisée à des températures comprises entre 25 et 44°C pendant 6 à 48h.

Une fraction du milieu d'enrichissement est ensuite prélevée dans le sac d'homogénéisation 10 et transférée lors de l'étape B, dans un conteneur secondaire, qui est ici constitué par un tube 20. Ce tube 20 comporte un milieu réactionnel 22. Ledit milieu réactionnel 22 peut être constitué d'un diluant (e.g. bouillon tryptone sel) apte à maintenir l'intégrité des microorganismes cibles et d'au moins un moyen de révélation. Le moyen de révélation peut être un colorant apte à colorer les microorganismes présents dans la fraction de milieu d'enrichissement transférée et issues de l'échantillon alimentaire 14. Le moyen de révélation peut également être un composé fluorescent rendant les microorganismes fluorescents. Dans le cas où l'on souhaite réaliser une subculture dans le tube 20, le milieu réactionnel pourra contenir en plus des nutriments, un système sélectif permettant d'accroitre la population de bactéries cibles.

Selon un exemple particulier, le moyen de révélation est basé sur la réduction du chlorure de triphényl 2-3-5-tétrazolium (TTC) par les microorganismes. Simultanément à la croissance des microorganismes, le TTC (incolore sous sa forme non réduite) est internalisé par lesdits microorganismes, puis réduit au sein du cytoplasme par ces derniers en triphényl-formazan (rouge) colorant ainsi lesdits microorganismes en rouge et permettant alors leur révélation sur le support. D'autres sels de tétrazolium peuvent être utilisés (CTC, MTT, etc...). En outre, des composés permettant d'accélérer la réaction de réduction des sels de tétrazolium peuvent être ajoutés au milieu réactionnel.

Il est également envisageable d'utiliser des colorants membranaires, tels que le violet de gentiane ou la fuchsine.

Dans le cas où le moyen de révélation est un composé fluorescent, il peut s'agir d'orangé d'acridine ou de diacétate de fluorescéine.

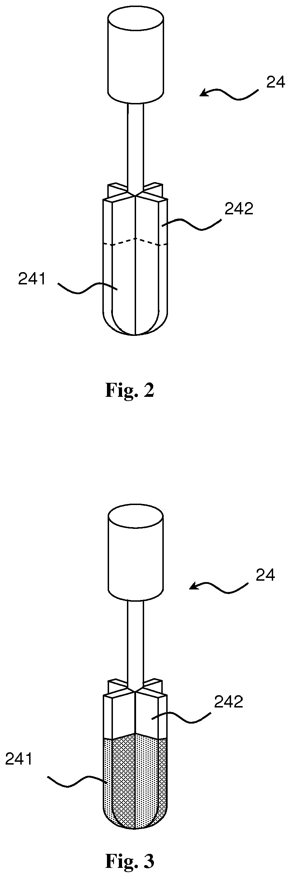

A l'étape C, un support de capture sensibilisé 24, est placé dans le tube 20 et est maintenu immergé dans le milieu réactionnel 22 par tout moyen approprié. Le support de capture sensibilisé 24 est fonctionnalisé par au moins un partenaire de liaison spécifique d'un microorganisme cible à détecter. Le support de capture peut être constitué de tout support apte à permettre la fixation de partenaires de liaison spécifique et bien connu de l'homme du métier. A titre d'exemple non limitatif, un support de capture adéquate peut être en polystyrène irradié, tel que celui commercialisé par la société Nunc/Thermo Scientific (Cat. No. 472230). Un tel support de capture est représenté schématiquement sur la figure 2, sous la référence 24. La partie inférieure peut être avantageusement et selon un mode de réalisation préférentiel, divisée en deux. La zone référencée 241 peut être sensibilisée avec une solution de partenaires de liaison (anticorps polyclonaux, anticorps monoclonaux, fragments Fab' ou Fab'2, aptamères, protéines de phage), alors que la partie supérieure 242 reste-elle vierge de tout partenaire de liaison et joue ainsi un rôle de contrôle négatif. Les techniques de sensibilisation de supports par des partenaires de liaison spécifiques sont bien connues de l'homme du métier.

Selon une alternative du procédé selon la divulgation il peut être avantageux de procéder à une étape de subculture, une fois le support de capture 24 plongé dans le milieu réactionnel 22. Cette subculture consiste à incuber le tube 20 pendant 1 à 18h, à des températures comprises entre 25 et 44°C. Selon cette alternative, les microorganismes sont colorés simultanément à leur croissance grâce au moyen de révélation contenu dans le milieu réactionnel. L'analyse peut alors se faire en temps réel.

Une fois la capture effective d'une certaine quantité de microorganismes cibles colorés ou fluorescents (cas d'un échantillon positif), il se produit un changement des propriétés optiques du support par apparition d'une coloration ou d'une fluorescence sur celui-ci (i.e. transduction du signal biologique). Cette coloration ou fluorescence du support de capture est alors détectable à l'œil ou mesurable via l'utilisation d'un automate de lecture tel qu'une caméra. Le support de capture est représenté schématiquement sur la figure 3, après analyse dont le résultat est positif. Comme on peut le voir, la zone 241 apparaît colorée du fait de la fixation des microorganismes cibles sur les partenaires de liaison spécifiques. La zone 242 jouant le rôle de témoin négatif demeure quant à elle, de la couleur initiale du support de capture.

Pour faciliter la lecture, il est préférable que le support de capture sensibilisé ne soit plus en contact avec le milieu réactionnel. A cette fin, il peut être envisagé par exemple de sortir le support de capture 24 dudit milieu réactionnel par tout moyen approprié, ceci est bien représenté à l'étape D, sur la figure 1. Comme explicité *supra,* la lecture peut être faite en point final, en pointillés ou en temps réel.

Selon une autre alternative du procédé selon la divulgation le support de capture est constitué par des particules sensibilisées, à savoir portant un partenaire de liaison spécifique ou non spécifique du ou des microorganisme(s) à détecter. La détection est alors préférentiellement mise en évidence par apparition d'une coloration ou fluorescence des particules sensibilisées initialement incolores, par l'intermédiaire des microorganismes cibles liés à ces dernières, au cours de la réaction.

Selon un mode de réalisation particulier, les particules sensibilisées peuvent être des particules magnétiques. Alors, la lecture peut se faire de façon manuelle et visuelle, en utilisant un système d'aimantation qui va permettre le rassemblement des particules magnétiques sur lesquelles sont capturés les microorganismes colorés ou fluorescents, sous forme d'amas contre la paroi du conteneur. Un déplacement du système d'aimantation verticalement vers le haut permet de sortir l'amas de particules magnétiques du milieu réactionnel et de faciliter ainsi l'analyse. Il peut être également envisagé de plonger le système d'aimantation directement dans le conteneur, afin de rassembler les particules magnétiques sur lesquelles sont fixées les microorganismes. Auquel cas, les particules magnétiques viennent se fixer directement sur le système d'aimantation qui devient alors coloré ou fluorescent.

Selon un autre mode de réalisation du procédé selon la divulgation, le milieu réactionnel et le support de capture, tels que décrits *supra* sont ajoutés directement dans le premier conteneur à l'issue de l'étape d'enrichissement. L'étape de détection est donc réalisée dans ledit premier conteneur sans transfert de tout ou partie du mélange constitué du milieu de culture et de l'échantillon à analyser vers un deuxième conteneur. Préalablement à cette étape de détection, une subculture peut être éventuellement réalisée afin d'accroitre la population de microorganismes cibles.

Il est à noter que, de façon avantageuse, le support de capture peut être protégé à l'aide d'un film protecteur. Ce film a pour but d'empêcher l'encrassement du support de capture. En effet, un tel encrassement est susceptible de diminuer les performances de capture dudit support. Un tel film peut être placé à demeure sur le support de capture, Alternativement, il peut s'agir d'un film qui a la capacité de se dissoudre au bout d'un certain temps de contact avec le milieu de culture liquide.

Le procédé selon l'invention est particulièrement avantageux car à l'issue de l'analyse, seuls les conteneurs identifiés comme positifs à l'aide du système de détection (décrit ci-dessous) sont ouverts afin de réaliser des analyses complémentaires de confirmation du résultat présomptif obtenu. L'étape de confirmation peut être effectuée au moyen d'une technologie différente de celle mise en œuvre dans le procédé selon l'invention.

Les exemples présentés ci-après ont pour but de présenter différents modes de réalisation du procédé selon l'invention et les résultats obtenus. Ils ne sont nullement limitatifs de l'invention.

### EXEMPLES

### Exemple 1: Détection optique de Salmonella Napoli, via l'utilisation d'un support sensibilisé, dans un échantillon alimentaire en subculture dans un milieu réactionnel

Le but de cette expérimentation est de détecter directement la présence de la bactérie cible *Salmonella* Napoli dans un échantillon alimentaire en subculture dans un milieu réactionnel, via l'utilisation d'un support sensibilisé, en polystyrène irradié, commercialisé par la société Nunc/Thermo Scientific (Cat. No. 472230) et représenté sur les figures 2 et 3.

Comme détaillé ci-après, la détection s'effectue pendant la phase réactionnelle en immergeant le support de capture sensibilisé avec une protéine de phage recombinante spécifique de *Salmonella* dans un tube qui contient l'échantillon enrichi, dilué au 1/100ème dans le milieu réactionnel.

### Protocole :

### Etape 1 : Remise en suspension des échantillons dans le milieu d'enrichissement primaire

Deux échantillons sont préparés comme suit :
Echantillon A : Dans un sac d'homogénéisation, 25g de fromage au lait cru contaminés par 5 unités formant colonies (UFC) de *Salmonella* Napoli sont remis en suspension dans 225 mL d'Eau Peptonée Tamponnée (EPT) (bioMérieux, Ref. 42043), supplémentés par 1 ml de Supplément SPT (bioMérieux, Ref 42650);
Echantillon B : Dans un sac d'homogénéisation, 25g de fromage au lait cru non contaminés par *Salmonella* Napoli sont remis en suspension dans 225 mL d'EPT (bioMérieux, Ref 42043) supplémentés par 1 mL de Supplément SPT (bioMérieux, Ref. 42650);

### Etape 2 : Après 16h d'incubation, transfert d'un aliquot de 0.1 mL du sac d'homogénéisation dans le tube réactionnel

0.1 mL du sac d'homogénéisation Echantillon A est transféré dans le tube réactionnel contenant 10 mL de SX2 (bioMérieux, Ref. 42121) et 1.6 g/L de TTC (bioMérieux, Cat. No. 04568088). On obtient ainsi l'Echantillon A'.

Une opération analogue est réalisée pour l'Echantillon B.

### Etape 3 : Immersion des supports sensibilisés dans les tubes réactionnels avant subculture et réaction

Le support de capture sensibilisé est disposé dans chaque tube (Echantillons A' et B'). Les tubes sont ensuite refermés et incubés dans une étuve à 37°C pendant 6 h.

### Etape 4 : Lecture des supports de capture à l'issue de la période d'incubation

Au terme de l'incubation (6h à 37°C) et suite à la réduction non spécifique du TTC par l'ensemble des bactéries présentes dans l'échantillon (i.e. appartenant à la flore annexe et la flore cible), le milieu réactionnel s'est coloré en rouge. Aussi, afin de pouvoir observer le support de capture révélant la positivité ou négativité de l'échantillon analysé, les tubes sont inclinés afin d'isoler ledit support de capture du milieu réactionnel.

Conformément au plan expérimental, le support de capture disposé dans l'échantillon A' apparaît coloré en rouge, confirmant que l'échantillon A' est positif alors que le support de capture disposé dans l'échantillon B' reste incolore, confirmant que l'échantillon B' est lui négatif. L'analyse de ces mêmes échantillons par la méthode VIDAS® SPT, commercialisée par la demanderesse (réf. 30707) a conduit à des résultats analogues, confirmant ainsi les résultats obtenus via lecture optique du support de capture sensibilisé.

### Exemple 2: Détection optique de Salmonella Napoli dans un échantillon biologique enrichi, via l'utilisation d'un support sensibilisé immergé dans un milieu réactionnel

Le but de cette expérimentation est de détecter directement la présence de la bactérie cible *Salmonella* Napoli dans un échantillon alimentaire enrichi, via l'utilisation d'un support sensibilisé, en polystyrène irradié, commercialisé par la société Nunc/Thermo Scientific (Cat. No. 472230) et représenté sur les figures 2 et 3.

Comme détaillé ci-après, la détection s'effectue pendant la phase réactionnelle en immergeant le support de capture sensibilisé avec une protéine de phage recombinante *anti-Salmonella* dans un tube qui contient l'échantillon enrichi, dilué au 1/2 dans le milieu réactionnel.

### Protocole :

### Etape 1 : Remise en suspension des échantillons dans le milieu d'enrichissement primaire

Deux échantillons sont préparés comme suit :
Echantillon A : Dans un sac d'homogénéisation, 25g de steak haché contaminés par *Salmonella* Napoli sont remis en suspension dans 225 mL d'EPT (bioMérieux, Ref. 42043) supplémentés par 1 mL de Supplément SPT (bioMérieux, Ref 42650);
Echantillon B : Dans un sac d'homogénéisation, 25g de steak haché non contaminés par *Salmonella* Napoli sont remis en suspension dans 225 mL d'EPT (bioMérieux, Ref 42043) supplémentés par 1 mL de Supplément SPT (bioMérieux, Ref 42650);

### Etape 2 : Après 16h d'incubation, transfert d'un aliquot de 1 mL du sac d'homogénéisation dans le tube réactionnel

1 mL du sac d'homogénéisation Echantillon A est transféré dans le tube réactionnel contenant 1 mL de tryptone sel (bioMérieux, Ref 42076) supplémenté par 10 µL de violet de gentiane (bioMérieux, Ref 55545). On obtient ainsi l'Echantillon A'.

Une opération analogue est réalisée pour l'Echantillon B.

### Etape 3 : Immersion des supports sensibilisés dans les tubes réactionnels avant réaction

Le support de capture sensibilisé est disposé dans chaque tube (Echantillons A' et B'), comme décrit ci-après. Les tubes sont ensuite refermés durant la période de réaction.

### Etape 4 : Lecture des supports de capture à l'issue de la période de réaction

Au terme de la réaction (40 min à température ambiante), l'ensemble des bactéries présentes dans l'échantillon (i.e. appartenant à la flore annexe et la flore cible) sont colorées en violet. Aussi afin de pouvoir observer le support de capture révélant la positivité ou négativité de l'échantillon analysé, les tubes sont inclinés afin d'isoler ledit support de capture du milieu réactionnel.

Conformément au plan expérimental, le support de capture disposé dans l'échantillon A' apparaît coloré en violet, confirmant que l'échantillon A' est positif alors que le support de capture disposé dans l'échantillon B' reste incolore, confirmant que l'échantillon B' est lui négatif. L'analyse de ces mêmes échantillons par la méthode VIDAS® SPT, commercialisée par la demanderesse (réf. 30707) a conduit à des résultats analogues, confirmant ainsi les résultats obtenus via une lecture à l'oeil du support de capture sensibilisé.

## Revendications

1. Procédé de détection, d'au moins un microorganisme présent dans un échantillon, ledit procédé comprenant essentiellement les étapes suivantes :
a) Mettre en contact dans un premier conteneur, ledit échantillon avec au moins un milieu de culture,
b) Placer ledit premier conteneur dans des conditions aptes à permettre la croissance du ou des microorganisme(s), pendant 6 à 48h ;
c) Mettre en contact tout ou partie du mélange constitué par l'échantillon et le milieu de culture avec un milieu réactionnel et un support de capture monobloc du ou desdit(s) microorganisme(s) dans un deuxième conteneur, ledit milieu réactionnel comportant des nutriments, un système sélectif permettant d'accroitre la population de bactéries cibles, un moyen de révélation du ou desdits microorganisme(s) choisi parmi les colorants et les composés fluorescents;
d) placer le deuxième conteneur dans des conditions aptes à permettre la croissance du ou des microorganisme(s).
e) Détecter à l'intérieur dudit deuxième conteneur, la présence du ou des microorganisme(s) révélé(s) par le moyen de révélation et fixé(s) sur le support de capture, la détection étant réalisée en temps réel.

2. Procédé selon l'une des revendications précédentes, comportant une étape supplémentaire consistant à confirmer la détection du ou des microorganismes détecté(s).

3. Procédé selon la revendication 2, dans lequel l'étape de confirmation est réalisée à l'aide d'un moyen de détection, identique ou différent du moyen de révélation utilisé pour la détection.

4. Procédé de détection selon l'une des revendications précédentes, dans lequel au moins un partenaire de liaison spécifique ou non spécifique du ou des microorganisme(s) est fixé sur le support de capture monobloc.

5. Procédé de détection selon la revendication précédente, dans lequel le partenaire de liaison spécifique est pris dans le groupe comprenant : les anticorps, les fragments Fab, les fragments Fab', les aptamères, les protéines de phages recombinantes ou non, les phages.

6. Procédé de détection selon l'une des revendications précédentes, dans lequel la détection du ou des microorganisme(s) est réalisée à l'issue de l'étape de croissance du ou desdits microorganisme(s).

7. Procédé de détection selon l'une des revendications précédentes, dans lequel le premier et/ou le deuxième conteneur est un sac d'homogénéisation, un flacon, une bouteille, un pilulier.

8. Procédé selon l'une des revendications précédentes, dans lequel le support de capture est un support monobloc poreux.

9. Procédé selon la revendication 8, dans lequel le support de capture est protégé par un film protecteur.

10. Procédé selon la revendication 8 ou 9, dans lequel le support de capture monobloc est un support compressible.

## Patentansprüche

1. Verfahren zum Nachweis mindestens eines Mikroorganismus, der in einer Probe vorliegt, wobei das Verfahren im Wesentlichen die folgenden Schritte umfasst:
a) Inkontaktbringen der Probe, in einem ersten Behälter, mit mindestens einem Kulturmedium,
b) Einwirkenlassen von Bedingungen, welche ein Wachstum des oder der Mikroorganismus/Mikroorganismen ermöglichen, auf den ersten Behälter über einen Zeitraum von 6 bis 48 Std.;
c) Inkontaktbringen, in einem zweiten Behälter, eines Teils oder der Gesamtheit der Mischung, welche aus der Probe und dem Kulturmedium besteht, mit einem Reaktionsmilieu und einem einstückigen Träger zum Abfangen des oder der Mikroorganismus/Mikroorganismen, wobei das Reaktionsmilieu Nährstoffe, ein selektives System, welches ein Wachstum der Population von Zielbakterien ermöglicht, ein Mittel zum Sichtbarmachen des oder der Mikroorganismus/Mikroorganismen umfasst, welches aus den Farbstoffen und den fluoreszierenden Verbindungen ausgewählt ist;
d) Einwirkenlassen von Bedingungen, welche ein Wachstum des oder der Mikroorganismus/Mikroorganismen ermöglichen, auf den zweiten Behälter;
e) Nachweisen, im Inneren des zweiten Behälters, des Vorhandenseins des oder der Mikroorganismus/Mikroorganismen, der/die durch das Mittel zum Sichtbarmachen sichtbar gemacht wurde(n) und auf dem Abfangträger fixiert wurde(n), wobei der Nachweis in Echtzeit erfolgt.

2. Verfahren nach einem der vorhergehenden Ansprüche, wobei es einen zusätzlichen Schritt aufweist, der darin besteht, den Nachweis des oder der nachgewiesenen Mikroorganismus/Mikroorganismen zu bestätigen.

3. Verfahren nach Anspruch 2, wobei der Schritt des Bestätigens mit Hilfe eines Nachweismittels durchgeführt wird, das bezüglich des Mittels zum Sichtbarmachen, welches für den Nachweis verwendet wird, gleichartig ist oder sich davon unterscheidet.

4. Nachweisverfahren nach einem der vorhergehenden Ansprüche, wobei an dem einstückigen Abfangträger mindestens ein Bindungspartner befestigt ist, der spezifisch oder unspezifisch für den oder die Mikroorganismus/Mikroorganismen ist.

5. Nachweisverfahren nach dem vorhergehenden Anspruch, wobei der spezifische Bindungspartner aus der Gruppe ausgewählt ist, die Folgendes umfasst: Antikörper, Fab-Fragmente, Fab'-Fragmente, Aptamere, Phagenproteine, die rekombinanter Art sind oder nicht, Phagen.

6. Nachweisverfahren nach einem der vorhergehenden Ansprüche, wobei der Nachweis des oder der Mikroorganismus/Mikroorganismen nach Abschluss des Schrittes des Wachstums des oder der Mikroorganismus/Mikroorganismen durchgeführt wird.

7. Nachweisverfahren nach einem der vorhergehenden Ansprüche, wobei es sich bei dem ersten und/oder dem zweiten Behälter um einen Homogenisierungsbeutel, ein Probenfläschchen, eine Flasche, eine Tablettenbox handelt.

8. Verfahren nach einem der vorhergehenden Ansprüche, wobei es sich bei dem Abfangträger um einen porösen einstückigen Träger handelt.

9. Verfahren nach Anspruch 8, wobei der Abfangträger durch eine Schutzfolie geschützt ist.

10. Verfahren nach Anspruch 8 oder 9, wobei es sich bei dem einstückigen Abfangträger um einen komprimierbaren Träger handelt.

## Claims

1. Method for detecting at least one microorganism present in a sample, said method essentially comprising the following steps:
a) in a first container, bringing said sample into contact with at least one culture medium,
b) placing said first container in suitable conditions to permit growth of the microorganism or microorganisms, for 6 to 48h;
c) bringing some or all of the mixture consisting of the sample and the culture medium into contact with a reaction mixture and a single-piece substrate for capturing said microorganism(s) in a second container, said reaction mixture comprising nutrients, a selective system allowing the population of target bacteria to increase, a detection mean for detecting the microorganism or microorganisms chosen among dyes and fluorescent compounds;
d) placing said second container in suitable conditions to permit growth of the microorganism or microorganisms,
e) detecting, within said second container, the presence of the microorganism or microorganisms detected by the detecting means and fixed on the capture substrate, the detection being carried out in real time.

2. Method according to one of the preceding claims, comprising an additional step consisting of confirming detection of the microorganism or microorganisms detected.

3. Method according to Claim 2, in which the confirmation step is carried out using a detecting means identical to or different from the detecting means used for detection.

4. Method of detection according to one of the preceding claims, in which at least one specific or non-specific binding partner of the microorganism(s) is fixed on the single-piece capture substrate.

5. Method of detection according to the preceding claim, in which the specific binding partner is taken from the group comprising: antibodies, Fab fragments, Fab' fragments, aptamers, recombinant or non-recombinant phage proteins, phages.

6. Method of detection according to one of the preceding claims, in which detection of the microorganism or microorganisms is carried out at the end of the step of growth of said microorganism(s).

7. Method of detection according to one of the preceding claims, in which the first and/or the second container is a homogenizing bag, a flask, a bottle or a tablet container.

8. Method according to one of the preceding claims, in which the capture substrate is a porous single-piece substrate.

9. Method according to Claim 8, in which the capture substrate is protected by a protective film.

10. Method according to Claim 8 or 9, in which the single-piece capture substrate is a compressible substrate.
